Europäisches Patentamt

European Patent Office

Office européen des brevets

(11) Publication number: **0 442 228 A1**

# EUROPEAN PATENT APPLICATION

(21) Application number: 90402171.4

(22) Date of filing: 27.07.90

(51) Int. Cl.5: **A61L 9/01**, A01N 59/20, F24F 3/16

(30) Priority: 16.01.90 JP 4471/90

(43) Date of publication of application:
21.08.91 Bulletin 91/34

(84) Designated Contracting States:
DE FR GB

(71) Applicant: MITSUBISHI JUKOGYO KABUSHIKI KAISHA
5-1, Marunouchi 2-chome Chiyoda-ku
Tokyo(JP)

(72) Inventor: Kinoshita, Tatsuyuki, C/o Nagoya Technical Inst.
Mitsubishi Jukogyo K.K., 1 Aza-Takamichi Iwatsuka-cho, Nakamura-ku, Nagoya, Aichi(JP)
Inventor: Takeuchi, Naokazu, C/o Nagoya Technical Inst.
Mitsubishi Jukogyo K.K., 1 Aza-Takamichi Iwatsuka-cho, Nakamura-ku, Nagoya, Aichi(JP)

(74) Representative: Rodhain, Claude et al
Cabinet Claude Rodhain 30, rue la Boétie
F-75008 Paris(FR)

(54) Deodorizing antibacterial composition.

(57) A deodorizing antibacterial composition, which comprises a combination of a cuprouscompound with an inorganic material based on solid acid or which comprises a dispersion or a solution of such composition in an aqueous solution of a reducing agent.

EP 0 442 228 A1

## DEODORIZING ANTIBACTERIAL COMPOSITION

The present invention relates to a deodorizing antibacterial composition and, in particular, to a composition capable of being used advantageously as a deodorizing antibacterial agent for air-conditioning, by incorporating in, for example, an air-conditioner or the like.

Hitherto, many deodorant products have found their practical uses. However, each of them exhibits particular disadvantages in eliminating the four essential odor origins, namely, ammonia, amines, hydrogen sulfide and mercaptans. In many cases, so-called physical adsorbents, such as, activated carbon, zeolites and so on, are employed in practice.

Use of these physical adsorbents may encounter problems such as follows:

1) Once adsorbed odoriferous compounds will be desorbed, when temperature and/or relative humidity of the environmental atmosphere increases, releasing thus again odors.

2) Upon deposition of dust and particles contained in the atmosphere on the adsorbent, microorganisms suspended in the air will find their growth bed thereon together with the adsorbed moisture and grow under emission of odoriferous metabolic products which form another odor origin.

Thus, the object of the present invention is to obviate the above problems of the deodorants of prior art and to provide a deodorizing antibacterial composition exhibiting an effective antibacterial activity, in addition to the superior deodorizing power.

The inventors had been engaged in sound researches for eliminating the above problems in the prior art and found that it was effective to employ a combination of a cuprous compound with an inorganic solid acid for the elimination of the existing problems, which has led to the completion of the present invention.

The gist of the present invention exists, in its first aspect, in (1) a deodorizing antibacterial composition comprising simultaneous existence of a cuprous compound and an inorganic solid acid and, in its second aspect, in (2) a deodorizing antibacterial composition comprising a dispersion or a solution of the above composition in an aqueous solution of a reducing agent.

According to the present invention, an effective and efficient removal of the four essential odor origins together with simultaneous attainment of exclusion of "re-release" of the once adsorbed odoriferous subtances, by combining an inorganic solid acid with a cuprous compound. It is able to attain by the present invention to eliminate simultaneously growth of microbes deposited on the adsorption bed from the atmospheric air. Moreover, the deodorizing antibacterial composition according to the present invention can be coated easily on various substrates of, such as, ceramics, metals, paper, plastics and so on, by formulating the composition in a form of a coating dispersion or coating solution in an adequate solvent, preferably water. Such substrates coated with the composition according to the present invention can be employed for uses for air-conditioning by installing them in, for example, air-conditioners and the like, as deodrizing element.

In the deodorizing antibacterial composition according to the present invention, the cuprous compound may be cuprous oxide or a cuprous salt such as cuprous chloride, as disclosed in the Japanese Patent Kokai No. 226357/1988 or a cuprous compound formed by adding a reducing agent such as L-ascorbic acid to a solution of a cupric salt such as cupric sulfate, as disclosed in the Japanese Patent Kokai No. 204755/1987. For the cupric salt, not only cupric sulfate, but also every other cupric salt including cupric nitrate, cupric acetate, cupric hydroxide and cupric oxide can be employed. For the reducing agent, there may be employed beside L-ascorbic acid other organic reducing agents, such as, erysorbic acid (iso-ascorbic acid) , oxalic acid and so on, inorganic reducing agents, such as, hydrogen, and further an electron transferring electrode reaction, in order to form corresponding cuprous compound.

For the inorganic solid acid, there may be enumerated. for example, silica, alumina, zeolites and various clay minerals.

For preparing the deodorizing antibacterial composition according to the present invention, the cuprous compound explained above and the inorganic solid acid mentioned above are mixed in a voluntary mixing ratio. While it is possible to bring the composition according to the present invention into practical use in a form of aqueous dispersion or solution, it is preferable to add to the composition a reducing agent, in order to prevent possible deterioration of the function of the cuprous compound by oxidation.

As the reducing agent, voluntary ones may be used, including, for example, ascorbic acid, as mentioned previously.

A coating composition can be prepared by adding to a slurry or an aqueous solution of the composition according to the present invention a binder, such as, colloidal silica, alumina sol, methylcellulose or so on. The so prepared coating composition can be applied on various substrates in a form of sheet, film, foil, net, foam or so on of paper, ceramics, metals, plastics and so on, by conventional application methods, such as, spraying, dipping, brushing and so on. After drying the resulting coated substrate, deodorizing antibacterial elements for incorporation in air-conditioner etc. are prepared.

The cuprous compound functions to remove odors by chemical reaction and oxidative decomposition of odor origins, such as, hydrogen sulfide, mercaptans and so on, avoiding thus "re-emission" of odoriferous substances. The cuprous compound reveals also an effect for preventing growth of microorganisms on the adsorption bed due to the function of cuprous ion and exhibits also a bactericidal activity due to the hydroxy radical ($\cdot$OH) formed by the reaction with oxygen.

The inorganic solid acid has a strong adsorbing power for basic odor origins, such as, ammonia and amines, by an ionic or a coordinative chemical bonding and brings about difficultly desorption or "re-release" of them. In this manner, the composition according to the present invention in which cuprous compound and an inorganic solid acid are employed in combination reveals a powerful action for removing the four essential odor origins.

Example 1

A pulverous mixture was prepared by mixing cuprous oxide and a zeolite of Y-form (HY) in a weight proportion of 1 : 1. Four vials filled each with the same air sample containing 1000 ppm of ammonia and each 100 ppm of trimethylamine, hydrogen sulfide and methyl mercaptan were charged each with 1 gram of the above pulverous mixture. After 30 minutes, 1 - 10 ml of each of the air samples in the vials were taken out and analyzed for the remaining concentration of the four odor substances by gas-chromatography. For the detecting device, TCD (thermal conductivity detector) for detecting ammonia, FID (hydrogen flame ionization detector) for detecting trimethylamine and FPD (flame photometric detector) for detecting hydrogen sulfide and methyl mercaptan were employed.

The results of the analysis after 30 minutes are given in Table 1, from which it is confirmed that the composition has a high effectiveness for removing the four essential odor origins, since all the analysis values for these origins lie below the detection limit.

Table 1

Effect of Removal of the Four Odor Origins

| Odor Origin | Concentration (ppm) | |
|---|---|---|
| | originally present | After 30 min. Treat. |
| Ammonia | 1,000 | 0 *) |
| Trimethylamine | 100 | 0 *) |
| Hydrogen Sulfide | 100 | 0 *) |
| Methyl Mercaptan | 100 | 0 *) |

*): Below the detection limit

Example 2

Using an aqueous dispersion prepared by dispersing each 20 % by weight of cuprous oxide and an aluminosilicate in a 1 % aqueous solution of ascorbic acid, a similar experiment as in Example 1 was carried out with the same result as above.

Example 3

A product of compacted granules having a grain size of 0.8 - 1.7 mm was prepared by pressing a pulverous mixture composed of cuprous oxide and a zeolite of Y-form in a weight mixing ratio of 1 : 1. 0.4 gram of this granule product was filled in a column of 8.1 mm ⌀, through which an air sample containing 10 ppm of hydrogen sulfide and 10 ppm of trimethylamine was passed until the concentration of each of these odor substances had reached at 5 ppm to determine the dynamic removal capacity of the column for these substances. For the sake of comparison, an activated carbon was tested in the same manner.

For the analysis, a batch-wise gas-chromatography with an FPD was employed for hydrogen sulfide and a continuous monitoring with a total hydrocarbon meter for trimethylamine. The results obtained are recited in Table 2.

## Table 2

## Comparison of Dynamic Removal Capacity

| Odor Origin | Dynamic Capacity (m-mol/g) | |
|---|---|---|
| | with $Cu_2O$ + HY | with Activ. Carbon |
| Hydrogen Sulfide | > 0.3 | 0.006 |
| Trimethylamine | > 0.4 | 0.04 |

Example 4

Each 200 parts by weight of cuprous oxide and an amorphous aluminosilicate ($Al_2O_3 \cdot 9SiO_2$) were dispersed in 800 parts by weight of a 1 % aqueous solution of ascorbic acid. An aqueous coating slurry was prepared by adding to the so obtained dispersion 20 % by weight of a colloidal silica. A deodorizing honeycomb element was prepared by coating a corrugated honeycomb profile (10 cm × 10 cm × 1 cm) made of a ceramic paper with this coating slurry by soaking.

In a testing duct constructed so as to permit to control the temperature and humidity of the air passing therethrough, the above honeycomb element was placed, in order to carry out a panel test evaluating the performance of the deodorizing element at a downstream portion of the duct by passing an air sample of low relative humidity containing 1 ppm of hydrogen sulfide at a flow rate of 1 m/sec for 1 hour and then changing over the passing air to that having a temperature of 25 °C and a relative humidity of 95 % without content of hydrogen sulfide at the same flow rate. For the sake of comparison, a commercial deodorizing honeycomb element coated with activated carbon fibers was employed and the same test was carried out. The results are summarized in Table 3.

## Table 3

## Results of Panel Test for Removal of Odor

| Degree of Odor in 6-Rank Evaluation | Honeycomb Element | |
|---|---|---|
| | with $Cu_2O$ + Al-silicate | with Activ. Carbon |
| Rank | 1 | 4 |

As seen in Table 3, the honeycomb element according to the present invention attained nearly complete deodorization, whereas the commercial honeycomb element showed strong leakage of the odor substance. Thus, it is confirmed that the deodorizing element according to the invention in which a cuprous compound and an inorganic solid acid are employed in combination does not cause desorption of the odor substance, whereas the deodorizing element of prior art shows a considerable leak or desorption of the odor substance.

Example 5

Deodorizing filters, which are prepared by impregnating a layer of polyurethane foam with a composition according to the invention composed of cuprous oxide and an amorphous aluminosilicate ($Al_2O_3 \cdot 9SiO_2 \cdot xH_2O$) under the use of an acrylic emulsion binder or by coating a layer of similar polyurethane foam with activated carbon, were tested for the performance of preventing growth of bacteria thereon together with a blank test using a similar filter layer of such polyurethane foam only.

These three test layers were placed each on an inorganic salt agar culture medium and thereon was sprayed a mixed spore dispersion prepared by dispersing spores of Aspergillus niger, Aspergillus terreus, Penicillium funiculosum, Paecilomyces variotti, Aureobasidium pullulens and Cladosporium cladosporioides in an aqueous aerosole solution having a content of sugar. The three samples each covered with lid were subjected to cultivation at 28° C for 2 weeks. The test results are given in Table 4, from which it is seen that no occurrence of hypha is recognized for the sample of the present invention, whereas the sample coated with activated carbon and the sample of blank test show growth of hypha over the entire surface of the sample, which indicates the antibacterial activity of the composition according to the present invention.

Table 4

Comparison of Antibacterial Activity

| Degree of Prevention according to JIS | Polyurethane Foam Filter Sample | | |
|---|---|---|---|
| | with Cu₂O + Al-silicate | with Activ. Carbon | Blank Test |
| Rank | 3 | · 1 | 1 |

Claims

1. A deodorizing antibacterial composition comprising a combination of a cuprous compound with an inorganic material based on solid acid.

2. A deodorizing antibacterial composition according to claim 1, characterized in that it is in a form of dispersion or solution in an aqueous solution of a reducing agent.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | WORLD PATENTS INDEX (LATEST), week 03, accession no. 90-019370, Derwent Publications Ltd, London, GB; & JP-A-1 299 558 (MITSUBISHI HEAVY IND. K.K.) 04-12-1989 * Abstract * | 1-2 | A 61 L 9/01 A 01 N 59/20 F 24 F 3/16 |
| X | WPI (LATEST), week 39, accession no. 89-282469, Derwent Publications Ltd, London, GB; & JP-A-1 207 116 (MITSUBISHI HEAVY IND. K.K.) 21-08-1989 * Abstract * | 1-2 | |
| X | CHEMICAL ABSTRACTS, vol. 109, no. 14, 3rd October 1988, page 321, abstract no. 115420w, Columbus, Ohio, US; & JP-A-63 132 661 (NIPPON ZEON CO., LTD) 04-06-1988 * Abstract * | 1-2 | |
| A | WPIL, week 30, accession no. 89-215641, Derwent Publications Ltd, London, GB; & JP-A-1 151 938 (MITSUBISHI HEAVY IND. K.K.) 14-06-1989 * Abstract * | 1-2 | |
| A | WPIL, week 51, accession no. 88-365304, Derwent Pubications Ltd, London, GB; & JP-A-63 275 799 (MITSUBISHI HEAVY IND. K.K.) 14-11-1988 * Abstract * | 1-2 | TECHNICAL FIELDS SEARCHED (Int. Cl.5) A 61 L |
| A | EP-A-0 260 869 (TAKEDA CHEMICAL INDUSTRIES, LTD) * Page 3, lines 11-24; claims 1-7 * | 1-2 | |

$-/-$

The present search report has been drawn up for all claims

| Place of search | Date of completion of search | Examiner |
|---|---|---|
| The Hague | 02 May 91 | ESPINOSA Y CARRETERO |

**European Patent Office**

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | US-A-4 797 274 (Y. MIKI et al.)<br>* Column 1, lines 42-68; column 2, lines 1-68 *<br>- - - - - | 1-2 | |

TECHNICAL FIELDS
SEARCHED (Int. Cl.5)

The present search report has been drawn up for all claims

| Place of search | Date of completion of search | Examiner |
|---|---|---|
| The Hague | 02 May 91 | ESPINOSA Y CARRETERO |

CATEGORY OF CITED DOCUMENTS

X : particularly relevant if taken alone
Y : particularly relevant if combined with another
document of the same catagory
A : technological background
O : non-written disclosure
P : intermediate document
T : theory or principle underlying the invention

E : earlier patent document, but published on, or after
the filing date
D : document cited in the application
L : document cited for other reasons

&: member of the same patent family, corresponding
document